# EUROPEAN PATENT SPECIFICATION

(11) **EP 1 550 434 B1**
(45) Date of publication and mention of the grant of the patent: **08.09.2010**
(21) Application number: 04030724.1
(22) Date of filing: 23.12.2004
(51) Int. Cl.: A61Q 5/12, A61K 8/37, A61K 8/39, A61K 8/89

(54) **Improved hair care compositions**
Verbesserte Haarpflegezusammensetzungen
Compositions de traitement capillaire ameliorées

(30) Priority: 30.12.2003 US 748737
(43) Date of publication of application: 06.07.2005
(73) Proprietor: AVON PRODUCTS, INC., Suffern NY 10901-5605 (US)
(72) Inventor: Robinson, Freda E., Nyack, New York 10960 (US); Duffy, John A., Mahwah, New Jersey 07430 (US); Buckridge, Kenneth., Mahwah, New Jersey 07430 (US)
(74) Representative: Vossius & Partner

(56) References cited:
- WO-A-02/22084
- WO-A-03/028680
- WO-A-03/028683
- US-A- 5 753 245
- US-A1- 2003 082 129
- US-A1- 2003 095 944
- US-A1- 2003 108 504
- US-A1- 2003 165 454
- US-A1- 2003 170 193
- US-B1- 6 432 393

## Description

This invention relates to hair care products. More particularly, it relates to rinse-off hair care products, such as shampoos, conditioning shampoos, and hair conditioners, which impart softness, smoothness, good combing and styling properties to both moist and dry hair. Most particularly, the present invention relates to shampoo and conditioning products that keep hair cleaner for a longer period of time while maintaining a smooth feel and a long-lasting conditioning effects thereon

Shampoo and conditioning products used in the normal course of bathing provide initial hair cleansing and hair conditioning benefits. Over time, the hair, however, there is a build-up of sebum from the scalp on the surface of the hair fibers. The sebum causes the hair to look and feel oily, and weighs the hair down. Accordingly, the hair loses its natural look and feel. Additionally, the sebum attracts dirt from the environment.

Accordingly, it would be advantageous if the hair shampoo and/or hair conditioning treatment provided a sebum reduction benefit, as well as an improved conditioning benefit, notwithstanding the removal of the hair treatment composition as a result of its use in the normal manner, i.e., as a rinse-off type of hair treatment product.

It has been found that shampoo and hair conditioning products containing a crosslinked silicone elastomer in combination with water soluble or water dispersible esters selected from polyethyleneglycol esters, polyglyceryl esters or sucrose esters, provide such improved conditioning benefits.

JP-A-6-80559 discloses a detergent composition that comprises a water-insoluble polymer powder (e.g., a silicone resin) and a water-insoluble, nonvolatile silicone (liquid or paste) dispersed in an aqueous solution of a surfactant. However, this composition fails to impart sufficient softness and moistening effects to the hair.

EP 855178 A2 discloses wash-off hair care products that include alpha-hydroxycarboxylic acids and water-insoluble silicone elastomer powders. The compositions of EP 855 178 A2 do not contain the esters of the present invention.

Accordingly, an object of the present invention is to provide hair care products that impart softness and smoothness to the moist hair, which properties are retained when the hair thus treated is dry.

A further object of the invention is to provide rinse-off hair care products that provide long-lasting conditioning effects.

Another object of the invention is to provide hair care compositions in which the cleansing benefit is not degraded over time as a consequence of sebum build-up.

Still a further object of the invention is provide a shampoo product that deters the build up of sebum on the surface of the hair, and that provides a conditioning benefit to the hair.

It is also an object of the invention to reduce irritation to the scalp by maintaining the hair in a cleaner state between shampooings.

These any other benefits of this invention will be more apparent upon reading of the detailed description of the Invention, a summary of which follows.

The compositions of the present invention are hair care products useful to impart a condition effect to the hair. The compositions may be shampoo products, conditioning shampoo products, or hair conditioning products, of the type that are applied to hair, left on the hair for a period of time, and then rinsed from the hair. The hair treatment compositions comprise a silicone elastomer, a water dispersible or water soluble ester, and a hair treatment active agent which may be selected, e.g., from the group consisting of surface active agents and hair conditioning agents.

The compositions of the present invention are improved compositions for application to any type of keratin fiber, e.g., hair, nails, eyebrows or eyelashes. They are preferably in non-emulsion form. Preferably, the compositions of present invention are hair care compositions, such as shampoos or conditioners. The compositions of the present invention include a synergistic combination of a solid crosslinked silicone elastomer and a water dispersible or water soluble ester. The compositions further contain at least one hair treatment active agent, which may be selected, e.g. from the group consisting of surface active agents and hair conditioning agents.

### Silicone Elastomer

Various solid crosslinked silicone elastomers may be used in the present invention. The solid silicone elastomers may be powders, and may comprise microspheres. SEM photographs of exemplary elastomers show that the silicone elastomers of the present invention have the requisite substantivity to adhere to the surface of a hair shaft. This provides benefits to the hair in several ways. First, damaged hair, that is, hair where the cuticle is broken, is sealed with a protective coating. Secondly, the elastomers attached to the hair provide a smoothness to the surface of the hair. Lastly, the sebum from the scalp is absorbed by the silicone spheres.

The crosslinked silicone elastomer is preferably formed from a polysiloxane polymer having at least two free reactive groups, preferably bonded to at least one terminal silicon atom. The reactive groups can include, but are not limited to, vinyl, allyl or epoxide moieties. The groups then react with Si-H linkages of another suitable polysiloxane backbone capable of participation in an addition reaction, such as a molecularly spherical MQ resin. The reaction results in a three-dimensional crosslinked polymer. The crosslinked silicone elastomer is essentially non-emulsifying (e.g., polyoxyalkylene groups absent). The weight average molecular weight of the crosslinked silicone elastomer preferably ranges from about 200,000 to about 20 million, most preferably from about 1 million to about 20 million.

Examples of crosslinked silicone elastomers suitable for use in the present compositions include, but are not limited to, one or more dimethicone crosspolymers, such as DC 9040 and DC 9042 Silicone Elastomer Blends sold by Dow Corning Corp.; organopolysiloxanes, for example, Polysilicone-11, a crosslinked siloxane rubber formed by reaction of vinyl-terminated siloxane and methylhydrodimethyl siloxane, such as Gransil DMG-6, Gransil GCM, Gransil PM-GEL and Gransil SR-5CYC sold by Grant Industries; dimethicone/vinyl dimethicone crosspolymers, such as Gransil RPS-NA sold by Grant Industries, SFE 839 sold by General Electric, KSG15, KSG 16 and KSG 17 sold by Shin-Etsu, and Dow Corning 9509 Silicone Elastomer Suspension sold by Dow Coming Corp.; dimethicone copolyol cross polymer; or any combinations thereof. Dimethicone/vinyl dimethicone crosspolymers for use in the present compositions include those disclosed in U.S. Patent Nos. 5,871,761 and 6,013,682. Most preferred examples of such useful crosslinked silicone elastomers are those provided in the form of non-ionic suspensions (for example, Dow Corning 9509 Silicone Elastomer Suspension of Dow Corning Corp. or non-ionic emulsions (for example, DC HMW 2220 Non-Ionic Emulsion sold by Dow Corning Corp.

The crosslinked silicone elastomer is solid. By the term "solid" it is meant that the crosslinked silicone elastomer does not flow or have a viscosity as do silicone resins. DC 9509 from Dow Corning Corp., discussed above, is an example of such a solid crosslinked silicone elastomer. Although the crosslinked silicone elastomer can be added directly to the hair composition, it is preferred that the crosslinked silicone elastomer is added to the hair composition in the form of pre-solubilized/pre-dispersed suspensions or emulsions.

The silicone elastomer used in the present invention is preferably a water-insoluble powder. It may be a composite powder comprising the above-described methylpolysiloxane or derivative thereof as the major component together with other water-insoluble polymer powder(s). Moreover, use may be made therefore of methylpolysiloxane or derivative thereof coated with a water-insoluble polymer by a conventional method. (Hereinafter, they will be sometimes referred to as "composite silicone elastomer powders.) The water-insoluble polymer as used herein includes polymethylsilsesquioxanes; amorphous silicon dioxide; polyethylene, polypropylene, polystyrene, polyamide, polyester or styrene/divinylbenzene copolymers.

The water-insoluble silicone elastomer powder has an average particle size of preferably from 0.01 to 100 µm, more preferably from 0.1 to 30 µm. The particles thereof may have a spherical, oval or spindle shape. Among these, spherical shape is preferable and particularly has a major axis/short axis ratio of 2 or below, preferably 1.3 or below.

It is preferable that the water-insoluble silicone elastomer powder has a true specific gravity of from 0.9 to 1.2, more preferably from 0.9 to 1.1. Further, it is preferable that the water-insoluble silicone elastomer powder has a rubber hardness (determined according to JIS K6253 using Type A Durometer) of from 5 to 95, more preferably from 10 to 90.

Although the water-insoluble silicone elastomer powder includes commercially available products such as KMP-598, KMP-597 and X-52-1139G (each manufactured by Shin-Etsu Chemical Co., Ltd.) and Trefil E-500, E-600, E-602, E-850 and E-730S (each manufactured by Dow Corning Toray Silicone Co., Ltd..), use may be made of either one of these water-insoluble silicone elastomer powder(s) or combination of two or more of the same.

The content of the silicone elastomer, e.g. in the form of a water-insoluble powder in the hair treatment compositions of the present invention preferably ranges on a weight basis, by weight of the total composition, from about 0.01 to 10%, more preferably from about 0.05 to 5%, more preferably from about 0.05 to 1.0 % by weight.

### Water Compatible Ester

The compositions of the present invention include at least one water dispersible or water soluble ester which may be an ethoxylated ester. It is believed that when the ester is incorporated into the water phase, it increases surface and application gloss. Preferred water dispersible or water soluble esters for use in the present invention include olive oil esters, polyethylene glycol esters, polyglycerol esters, and silicone esters. As a measure of water dispersibility and water solubility, the esters of the present invention should have a hydrophilic/lipophilic balance (HLB) of 8.5 or above, preferably about 10.0 and above, especially between about 10 and 18, and most preferably between about 10.5 and 16.

Esters useful in a composition according to the present invention include olive oil PEG-7 esters. A preferred olive oil PEG-7 ester is sold by Seppic, Inc., Paris, France, under the tradename Olivem 300. Another useful ester of this type is PEG-7 glyceryl cocoate sold, e.g., by Cognis GmbH under the tradename cetiol HE. Polyethylene glycol esters are useful in the compositions according to the present invention, especially such esters derived from glycerides and such esters derived from fatty acids and having a degree of propoxylation. A preferred PEG-9 cocoglyceride is sold by Inolex Chemical Co., Philadelphia, PA, under the tradename Lexol EC. Another suitable ester is PEG-6 Caprylic/Capric Glycerides sold under the tradename Glycerox 767 by Croda. Preferred esters derived by reaction of a fatty acid with ethylene oxide and propylene oxide are PEG/PPG-8/3 diisostearate and PEG/PPG-8/3 laurate sold by Scher Chemicals, Inc., Clifton, NJ. Polyglycerol esters useful in a composition according to the present invention include polyglycerol-3 laurate, triglycerol laurate and glycereth-26 trioctanoate. A preferred polyglycerol-3 laurate ester is sold by Scher Chemicals, Inc., Clifton, N.J., under the tradename Hydramol TGL. A preferred glycereth-26 trioctanoate is sold by Heterene, Inc., Paterson, N.J., under the tradename Hest G-26-TO. Also suitable are esters based on sucrose, such as sucrose laurate, sucrose palmitate and sucrose stearate. Such esters may be mixed mono and diesters, provided that the monoester content of the ester is sufficient to provide the requisite HLB value. Sucrose esters that are suitable include Sistema L70-C (sucrose laurate with 70 % monoester content); Sistema PS750-C (sucrose palmitate with 75 % monoester content), and Sistema SP50-C (sucrose stearate with 50 % monoester content);

The water compatible ester is preferably present in an amount about 0.1 wt % to about 20 wt %, preferably in an amount about 0.25 wt% to about 5 wt %, more preferably from about 0.25 to 2.5 wt %, most preferably from about 0.25 to about 1 wt %, based on the total weight of the composition.

### Surfactants

The hair care product of the present invention may further contain surfactant(s), and the addition of at least one surfactant is particularly preferred when the composition is a hair care composition. These surfactants are not particularly restricted but arbitrarily selected from those commonly employed in cosmetics.

The surfactants that may be included in the compositions of the present invention are selected from the group consisting of anionic, nonionic, cationic, amphoteric, zwitterionic, and ampholytic surfactants. The choice of surfactants depends on the type of product that is being made. Thus, anionic, nonionic and amphoteric surfactants are generally incorporated in shampoo product, while catioinic and amphoteric surfactants, generally provided as quaternary ammonium compounds, are often present in hair conditioning products, including conditioning shampoo products that provide the dual utility of cleansing and conditioning the hair.

Surfactants suitable for use as cleansing agents are listed in the International Cosmetic Ingredient Dictionary and Handbook, 9th Edition, 2002, Vol. 4, page 2955 et seq. Hair conditioning agents are identified at page 2912, with quaternary ammonium compounds being set forth at page 2957.

For the hair conditioning composition, the content of the cationic, amphoteric, zwitterionic and ampholytic surfactant(s) in the composition preferably ranges from about 0.1 to about 20% by weight, in particular, about 0.5 to about 10% by weight, based on the whole composition.

For the shampoo compositions, the content of anionic and nonionic surfactant(s) in the composition preferably ranges from about 5 to about 30 % by weight, in particular, about 10 to about 25 % by weight, based on the whole composition.

In addition to the components described above, the hair care products of the present invention may optionally contain components commonly employed in cosmetics, such as oily components such as hydrocarbon oils, vegetable oils, fatty acids, ester oils, perfluoropolyethers and silicone derivatives; medicinal components such as antidandruff agents, bactericides and vitamins; preservatives such as paraben; thickeners such as water-soluble polymers, coloring matters such as dyes and pigments; conditioning agents such as cationic polymers; pearling agents; and those listed in Encyclopedia of Conditioning Rinse Ingredients (Micelle Press, 1987), so long as the effects of the present invention are not deteriorated thereby.

The hair care products of the present invention can be produced by blending the components in a conventional manner. The hair care products of the present invention are preferably in the form of products to be washed away after using, for example, shampoo compositions such as shampoos and rinse-in-shampoos, rinses, hair treatments, hair conditioners and hair packs.

### Effect of the Invention

The hair care products of the present invention can impart softness, smoothness and good combing properties to the moist hair and, after drying, make the hair soft, moist and well styled while keeping smooth or oil-free feel and exerting long-lasting conditioning effects thereon. In particular, the present invention provides compositions that allow hair to feel cleaner longer. Generally, the scalp excretes sebum and the sebum wicks up the shaft of the hair, thereby creating a feeling of dirty weighed down hair. The present invention prevents the wicking of the sebum up the shaft of the hair, thereby allowing the hair to be and feel cleaner for a longer period of time. It has also been found that the specific combination of the esters of the present invention with the silicone elastomers provides improved wet and dry hair properties. In particular, the smoothness and combing properties noticed in respect of wet hair are also manifest after the hair has been dried.

The present invention also provides a method for absorbing and/or removing sebum and/or styling product residue from the hair or scalp. The method comprises the step of applying the water-based composition to the hair and/or scalp. Preferably, the water-based hair composition is distributed through the hair and/or scalp, e.g., by using a comb or brush.

### Optional Ingredients

Compositions of the present invention may also contain one or more additional ingredients conventionally incorporated into hair care compositions. Such additional ingredients include styling agents, such as resins and hair-setting polymers, perfumes, dyes, buffering or pH adjusting agents, viscosity modifiers, opacifiers, pearlizers, preservatives, antibacterial agents, antidandruff agents, vitamins, foam boosters, proteins, moisturizing agents, herb or other plant extracts, or other natural ingredients. The compositions of the present invention may include one or more additional components, such as one or more antimicrobials, antioxidants, buffering agents, chelating agents, colorants, conditioning agents, emollients, film formers, fragrances, humectants, lubricants, moisturizers, pigments, preservatives, skin penetration enhancers, stabilizers, vitamins, or any combinations thereof.

### Product Forms

The compositions of the present invention may have a number of different product forms. Such suitable product forms include an aerosol or pump spray, mousse, foam, solution, serum, or the hair care composition may be incorporated into a towelette. Most preferably, the composition is in a product form that allows for easy transportation, access and use. Most preferably the product form is a sprayable (e.g., aerosol or pump sprays) solution. The compositions of the present invention may be incorporated into combs and brushes within a separate reservoir in the same manner used for dental care toothbrushes that dispense dentifrices and toothpastes. Examples of such systems are disclosed in U.S. Patents No. 5913632; 5882134; 4787765; 6206600; and 6257791.

In preferred form, the present invention is provided in a small package that can be conveniently carried in a pocket, handbag or briefcase.

### EXAMPLES

Examples of compositions made according to the present invention may be made according to the following.

### Example 1

| Shampoo Composition | Weight % (Range) |
|---|---|
| Water | 30-84 |
| Sodium polyoxyethylene lauryl ether sulfate (SLES) | 10.0-35.0 |
| Sodium polyoxyethylene lauryl sulfate (SLS) | 4.0-15.0 |
| Silicone Elastomer* | 0.1-3.0 |
| Triglycerol Laurate | 0.1-5.0 |
| Guar HPT Chloride | 0.05-2.0 |
| Cocamide MEA | 1.0-5.0 |
| Phosphoric Acid | 0.01-.25 |
| Cocamidopropylbetaine | 1.0-8.0 |

| | |
|---|---|
| * Dow Corning 9509 Silicone Elastomer Suspension (61.4 % active) | |

### Example 2

| Shampoo Composition | Weight % (Range) |
|---|---|
| Water | 30-84 |
| Sodium polyoxyethlene lauryl ether sulfate (SLES) | 10.0-35.0 |
| Sodium polyoxyethlene lauryl sulfate (SLS) | 4.0-15.0 |
| Silicone Elastomer* | 0.1-3.0 |
| Sucrose ester (40 % active)* | 0.25-5.0 |
| Guar HPT Chloride | 0.05-2.0 |
| Cocamide MEA | 1.0-5.0 |
| Phosphoric Acid | 0.01-.25 |
| Cocamidopropylbetaine | 1.0-8.0 |

| | |
|---|---|
| * Dow Corning 9509 Silicone Elastomer Suspension (61.4 % active) ** Sisterna L70-C | |

### Example 3

| Shampoo Composition | Weight % (Range) |
|---|---|
| Water | 30-84 |
| Sodium polyoxyethlene lauryl ether sulfate (SLES) | 10.0-35.0 |
| Sodium polyoxyethlene lauryl sulfate (SLS) | 4.0-15.0 |
| Silicone Elastomer* | 0.1-3.0 |
| PEG/PPG-8/3 laurate | 0.1-2.0 |
| Guar HPT Chloride | 0.05-2.0 |
| Cocamide MEA | 1.0-5.0 |
| Phosphoric Acid | 0.01-.25 |
| Cocamidopropylbetaine | 1.0-8.0 |

| | |
|---|---|
| * Dow Corning 9509 Silicone Elastomer Suspension (61.4 % active) | |

### Example 4

Comparative tests were conducted to evaluate a shampoo compositions of the present invention against similar compositions, but not containing either or both of the silicone elastomer and the ethoxylated ester. Each of the compositions tested were evaluated for wet detangling, wet feel after wet detangling, wet combing, wet feel after wet combing, dry detangling, and dry feel.

A shampoo base composition was prepared, which did not contain either a silicone elastomer or an ethoxylated ester, and is tabulated below:

### Base Composition

| Component | Weight (grams) |
|---|---|
| Demineralized water | qs 98 grams |
| Anionic surfactant blend (56.5 % active) | 20.75 |
| Sultaine (50% active) | 4.0 |
| Alkanolamide | 2.8 |
| Polyquaternium 44 (7% active) | 0.5 |
| Protein | 0.2 |
| Quaternized polymer | 0.4 |
| Fragrance | 0.5 |
| Chelating agent | 0.2 |
| pH modifier | 0.12 |
| Preservative | 0.6 |
| Miscellaneous adjuvants | 0.175 |

Composition A (the Control) was prepared by the addition of 2 g. water to the Base Composition. Composition B was prepared by adding to the Base Composition sufficient silicone elastomer to provide an elastomer concentration of 0.46 wt% and water qs 100 g. No ethoxylated ester was added to Composition B. Composition C was prepared by adding to the Base Composition sufficient ethoxylated ester to provide an ester concentration of 0.35 wt% and water qs 100 g. No elastomer was added to Composition C. Composition D (this Invention) was prepared by adding to the Base Composition sufficient sufficient silicone elastomer to provide an elastomer concentration of 0.46 wt% and sufficient ethoxylated ester to provide an ester concentration of 0.35 wt%. The silicone elastomer used in each composition was 0.75 % Dow Corning 9509 Silicone Elastomer Suspension, which is 61.4 % active by weight to yield a 0.46 wt% active level of the elastomer. The esters used were (1) Polyglyceryl-3 Laurate (provided as Hydramol TGL); (2) PEG-7 Glyceryl Cocoate (provided as Glycerox HE); (3) PEG/PPG-8/3 Laurate (provided as Hydramol PGPL), and (4) sucrose laurate (provided as Sistema L70-C, which is 40 wt % active). The Sistema L70-C sucrose ester was incorporated into the Base Composition on an "as is" basis. For example, Compositions C and D of this Example 4 contained 0.35 wt % Sistema L70-C, which is equivalent to 0.14 wt % sucrose laurate on an actives basis. The other esters are 100 % active.

European hair tresses were treated as follows. 1.0 g of a shampoo composition as described above was applied to the human hair tress (weight of tress about 15 g; length about 22.5 cm). After foaming shampoo for approximately one minute, the tress was rinsed with running water for 60 seconds and excess water then removed by patting with a paper towel.

The treated tresses with the compositions described above were evaluated blindly by 3 panelists (unless otherwise noted) trained in the evaluation of such hair tresses, in each of the categories mentioned above. The tresses were evaluated with the large end of a standard comb to detangle and the small end of the standard comb for wet and dry combing. Wet and dry feel were ranked after combing. For each test category, the panelist rated the tress with a number from 1 to 4, with the number 1 being "best" and the number "4" being worst, for each of the four compositions A through D. The number of points received by each test composition was totaled in each test category, and also overall, with the lowest score in each category (and overall) being the best performing product in the category (and overall). Conversely, the highest scores represented the poorest performing product. The results are tabulated below.

**Table 1**

| Ester is Polyglyceryl-3 Laurate (1)* | | | | | | | |
|---|---|---|---|---|---|---|---|
| Composition | Wet Detangling | Wet Feel (after detangling) | Wet Combing | Wet Feel (after combing) | Dry Detangling ** | Dry Feel ** | Cumm. Score |
| A | 11 | 12 | 11 | 12 | 6 | 8 | 60 |
| B | 4 (2) | 4 (2) | 5 (2) | 6 | 5 | 4 | 28 (2) |
| C | 9 | 9 | 9 | 9 | 7 | 6 | 49 |
| D | 6 (1) | 5 (1) | 5 (1) | 3 (3) | 2 (2) | 2 (2) | 23 (10) |

| | | | | | | | |
|---|---|---|---|---|---|---|---|
| * First place ratings in parentheses. * Partial panel. | | | | | | | |

**Table 2**

| Ester is PEG-7 Glyceryl Cocoate (2)* | | | | | | | |
|---|---|---|---|---|---|---|---|
| Composition | Wet Detangling | Wet Feel (after detangling) | Wet Combing | Wet Feel (after combing) | Dry Detangling | Dry Feel | Cumm. Score |
| A | 9 | 10 | 11 | 7 (1) | 11 | 12 | 60 (1) |
| B | 5 (2) | 7 (1) | 10 | 10 | 10 | 9 | 41 |
| C | 9 | 5 (2) | 4 (2) | 5 (2) | 5 (1) | 4 (2) | 32 (9) |
| D | 7 (1) | 8 | 5(1) | 8 | 4 (2) | 5 (1) | 37 (5) |

| | | | | | | | |
|---|---|---|---|---|---|---|---|
| *First place ratings in parentheses. | | | | | | | |

**Table 3**

| Ester is PEG/PPG-8/3 Laurate (3)* | | | | | | | |
|---|---|---|---|---|---|---|---|
| Composition | Wet Detangling | Wet Feel (after detangling) | Wet Combing | Wet Feel (after combing) | Dry Detangling** | Dry Feel** | Cumm. Score |
| A | 8 (1) | 9 | 8 | 12 | 3 | 6 | 46 (1) |
| B | 9 | 9 | 9 | 9 | 4 | 5 | 45 |
| C | 5(2) | 8(1) | 9 | 6 | 1 (1) | 4(1) | 33(5) |
| D | 8 | 4(2) | 3(3) | 3(3) | 2 | 5(1) | 25(9) |

| | | | | | | | |
|---|---|---|---|---|---|---|---|
| * First place ratings in parentheses. ** Partial panel. | | | | | | | |

**Table 4**

| Ester is Sucrose Laurate Sisterna L70-C (4)* | | | | | | | |
|---|---|---|---|---|---|---|---|
| Composition | Wet Detangling | Wet Feel (after detangling) | Wet Combing *** | Wet Feel (after combing) | Dry Detangling | Dry Feel | Cumm. Score |
| A | 8 | 10 | 9 | 9 | 10 | 12 | 58 |
| B | 10 | 10 | 9 | 11 | 8 | 5 (1) | 53 (1) |
| C | 9 | 6 (1) | 9 | 7 | 8 (1) | 9 | 48 (2) |
| D | 3 (3) | 4 (2) | 4 (2) | 3 (3) | 4 (2) | 4 (2) | 22 (14) |

| | | | | | | | |
|---|---|---|---|---|---|---|---|
| * First place ratings in parenthesis. *** Four second places; two first places. | | | | | | | |

The evaluations above confirm that the compositions of the present invention provide superior conditioning performance.

### Example 5

The tests of Example 4 were repeated, but at one-half of the elastomer and ethoxylated ester levels, that is, with 0.23 wt% of the silicone elastomer (0.375 % Dow Cornng 9509 Silicone Elastomer Suspension, which is 61.4 % active by weight),and 0.175 wt% of one of the ethoxylated esters designated below. As in Example 4, Compositions C and D contained Sistema L70-C as received.

**Table 5**

| Ester is PEG-7 Glyceryl Cocoate (2)* | | | | | | | |
|---|---|---|---|---|---|---|---|
| Composition | Wet Detangling | Wet Feel (after wet detangling) | Wet Combing | Wet Feel (after wet combing) | Dry Detangling | Dry Feel | Cumm. Score |
| A | 6 (1) | 9 | 8 (1) | 9 | 6 | 10 | 48 (2) |
| B | 7 | 8 | 7 | 10 | 5 (2) | 9 | 46 (2) |
| C | 11 | 9(1) | 7(1) | 8 | 10 | 8 | 53(2) |
| D | 6 (2) | 4 (2) | 8 (1) | 3 (3) | 9(1) | 3 (3) | 33 (12) |

| | | | | | | | |
|---|---|---|---|---|---|---|---|
| * First place ratings in parentheses. | | | | | | | |

**Table 6**

| Ester is PEG/PPG-8/3 Laurate (3)* | | | | | | | |
|---|---|---|---|---|---|---|---|
| Composition | Wet Detangling | Wet Feel (after detangling) | Wet Combing | Wet Feel (after combing) | Dry Detangling** | Dry Feel** | Cumm. Score |
| A | 10 | 10 | 9(1) | 9 (1) | 3 (1) | 4 | 45 (3) |
| B | 5(2) | 5(2) | 10 | 7 | 5(1) | 1(1) | 33(6) |
| C | 7 (1) | 9 | 7 | 7 | 7 | 3 | 40 (1) |
| D | 7 | 6(1) | 4(2) | 4(2) | 5 | 2 | 28(5) |

| | | | | | | | |
|---|---|---|---|---|---|---|---|
| * First place ratings in parentheses. * Partial panel. | | | | | | | |

**Table 7**

| Ester is sucrose laurate Sistema L70-C (4)* | | | | | | | |
|---|---|---|---|---|---|---|---|
| Composition | Wet Detangling | Wet Feel (after detangling) | Wet Combing | Wet Feel (after combing) | Dry Detangling** | Dry Feel** | Cumm. Score |
| A | 9 | 7 (1) | 8 (1) | 8 | 6 | 5 | 43 (2) |
| B | 11 | 11 | 11 | 10 | 2 (2) | 8 | 53 (2) |
| C | 7 | 8 | 6 | 9 | 6 | 5 | 41 |
| D | 3 (3) | 4 (2) | 5(2) | 3 (3) | 5 | 2 (2) | 22 (12) |

| | | | | | | | |
|---|---|---|---|---|---|---|---|
| * First place ratings in parenthesis. * Partial panel. | | | | | | | |

The evaluations above confirm that the compositions of the present invention provide superior conditioning performance.

### Example 6

The tests of Example 4 were repeated, but at twice the elastomer and ethoxylated ester levels, that is, with 0.92 wt% of the silicone elastomer (1.5 % Dow Cornng 9509 Silicone Elastomer Suspension, which is 61.4 % active by weight) and 0.70 wt% of one of the ethoxylated esters designated below. As in Example 4, Compositions C and D contained Sistema L70-C as received.

**Table 8**

| Ester is PEG-7 Glyceryl Cocoate (2)* | | | | | | | |
|---|---|---|---|---|---|---|---|
| Composition | Wet Detangling | Wet Feel (after wet detangling) | Wet Combing | Wet Feel (after wet combing) | Dry Detangling | Dry Feel | Cumm. Score |
| A | 8(1) | 10 | 6(1) | 5(1) | 10 | 10 | 49(3) |
| B | 11 | 9 | 7(1) | 12 | 3 (3) | 5 (2) | 47 (6) |
| C | 6 (1) | 5 (1) | 10 | 9 | 9 | 9 | 48 (2) |
| D | 5 (1) | 6(2) | 7 (1) | 4 (2) | 8 | 6 (1) | 36 (7) |

| | | | | | | | |
|---|---|---|---|---|---|---|---|
| * First place ratings in parentheses. | | | | | | | |

**Table 9**

| Ester is PEG/PPG-8/3 Laurate (3)* | | | | | | | |
|---|---|---|---|---|---|---|---|
| Composition | Wet Detangling | Wet Feel (after detangling) | Wet Combing | Wet Feel (after combing) | Dry Detangling** | Dry Feel** | Cumm. Score |
| A | 7(1) | 10 | 9(1) | 9(1) | 3(1) | 4 | 45(3) |
| B | 5(2) | 5(2) | 10 | 7 | 5(1) | 1 (1) | 33(6) |
| C | 7 (1) | 9 | 7 | 7 | 7 | 3 | 40 (1) |
| D | 7 | 6 | 4(2) | 4(2) | 5 | 2 | 28(4) |

| | | | | | | | |
|---|---|---|---|---|---|---|---|
| * First place ratings in parentheses. * Partial panel. | | | | | | | |

**Table 10**

| Ester is sucrose laurate Sistema L70-C (4)* | | | | | | | |
|---|---|---|---|---|---|---|---|
| Composition | Wet Detangling | Wet Feel (after detangling) | Wet Combing | Wet Feel (after combing) | Dry Detangling** | Dry Feel** | Cumm. Score |
| A | 9 (1) | 9 (1) | 8 | 7 (1) | 6 | 5 | 44 (3) |
| B | 4(2) | 5(1) | 7(1) | 11 | 6 | 5(1) | 38(5) |
| C | 10 | 10 | 11 | 7 | 6 | 4 (1) | 48 (1) |
| D | 7 | 9 | 4(2) | 5(2) | 4(1) | 4 | 33(5) |

| | | | | | | | |
|---|---|---|---|---|---|---|---|
| * First place ratings in parenthesis. * Partial panel. | | | | | | | |

The evaluations above confirm that the compositions of the present invention provide superior conditioning performance.

It should be understood that the foregoing description is only illustrative of some embodiments of the present invention. Various alternatives and modifications can be devised by those skilled in the art without departing from the invention. Accordingly, the present invention is intended to embrace all such alternatives, modifications and variances that fall within the scope of the appended claims.

## Claims

1. A rinse-out non-emulsion hair-care composition comprising
(a) a solid crosslinked silicone elastomer,
(b) a waster dispersible or water soluble ester selected from polyethyleneglycol esters, polyglyceryl esters and sucrose esters,
(c) at least one hair treatment active agent
(d) at least about 20 wt% water.

2. The hair care composition according to claim 1, wherein the solid silicone elastomer is present in an amount effective to deposit a sebum-absorbing level of said crosslinked silicon elastomer on the hair or scalp after the composition has been applied to the hair or scalp.

3. The hair care composition according to claim 1 or 2, wherein the silicone elastomer is a powder.

4. The hair care composition according to any of claims to 3, wherein the silicone elastomer comprises microspheres.

5. The hair care composition according to any of claim 1 to 4, wherein the silicone elatomer has a weight average molecular weight from 1 to 20 million.

6. The composition of any of claims 1 to 5, wherein said silicone elastomer is a dimethicone crosspolymer, an organopolysiloxane, a dimethicone/vinyl dimethicone crosspolymer, a dimethicone copolyol cross polymer, or any combination thereof.

7. The composition of any of claims 1 to 5, wherein the silicone elastomer is the reaction product of a polysiloxane having at least two free reactive groups from vinyl, alkyl and epoxide groups and a polysiloxane having an Si-H linkage.

8. The composition of any of claims 1 to 7, wherein the hair treatment active agent comprises at least one surfactant selected from the group consisting of anionic, nonionic, cationic, amphoteric, zwitterionic, and ampholytic surfactantants.

9. The composition of any of claims 1 to 7, wherein the hair treatment active agent comprises a hair conditioning agent.

10. The composition of any of claims 1 to 9, wherein the water dispersible or water soluble ester is present in an amount of from about 0.1 to 20 wt%, based on the total weight of the composition, the ester having an HLB of about 8.5 and above.

11. The composition of any of claims 1 to 10, wherein the HLB of the water dispersible or water soluble ester is about 10 and above.

12. The composition of any of claims 1 to 11, wherein the ester is selected from the group consisting of PEG-7 oliveate, PEG-7 glycerol cocoate, PEG-9 cocoglyceride, PEG/PPG-8/3 laurate, PEG/PPG-8/3 diisostearate, triglycerol laurate, gtycereth-26 trioctanoate, polyglycerol-3 laurate, sucrose laurate, sucrose palmitate, and sucrose stearate.

13. The composition of claim 11, wherein the ester is selected from the group consisting of PEG-7 glyceryl cocoate, PEG/PPG-8/3 laurate, PEG/PPG-8/3 diisostearate, triglycerol laurate, and sucrose stearate.

14. The composition of any of claims 1 to 13, wherein the water dispersible or water soluble ester is present in an amount of from about 0.1 to about 5 wt %.

15. The composition of any of claims 1 to 14, wherein the silicone elastomer is present in an amount of from about 0.01 to about 5 wt%, by weight of the total composition.

16. The composition of any of claims 1 to 15, comprising from about 5 to about 30 wt % of a surfactant selected from the group consisting of anionic and nonionic surfactants.

17. The composition of any of claims 1 to 16, wherein the composition is a shampoo.

18. The composition of any of claims 1 to 15, comprising from about 0.1 to about 20 wt%, of a surfactant selected from the group consisting of cationic, amphoteric and zwitterionic surfactants.

19. The composition of any of claims 1 to 15 or 18, wherein the composition is a hairconditioner.

20. A method of treating hair comprising:
applying to the hair a composition according to any of claims 1 to 19.

## Patentansprüche

1. Eine Haarpflegezusammensetzung zum Auswaschen, die keine Emulsion ist, umfassend:
(a) ein festes vernetztes Siliconelastomer,
(b) einen in Wasser dispergierbaren oder in Wasser löslichen Estern, ausgewählt aus Polyethylenglycolestern, Polyglycerylestern und Sucroseestern,
(c) mindestens einen Wirkstoff zur Haarbehandlung,
(d) mindestens etwa 20 Gew.-% Wasser.

2. Die Haarpflegezusammensetzung gemäß Anspruch 1, wobei das feste Siliconelastomer in einer Menge vorhanden ist, die eine Sebum absorbierende Menge des vernetzten Siliconelastomers auf dem Haar oder der Kopfhaut wirksam ablagert, nachdem die Zusammensetzung auf das Haar oder die Kopfhaut aufgetragen wurde.

3. Die Haarpflegezusammensetzung gemäß Anspruch 1 oder 2, wobei das Siliconelastomer ein Pulver ist.

4. Die Haarpflegezusammensetzung gemäß einem der Ansprüche 1 bis 3, wobei das Siliconelastomer Mikrokügelchen umfasst.

5. Die Haarpflegezusammensetzung gemäß einem der Ansprüche 1 bis 4, wobei das Siliconelastomer ein gewichtsgemitteltes Molekulargewicht von 1 bis 20 Millionen aufweist.

6. Die Zusammensetzung nach einem der Ansprüche 1 bis 5, wobei das Siliconelastomer ein Dimethicon-Crosspolymer, ein Organopolysiloxan, ein Dimethicon-/Vinyldimethicon-Crosspolymer, ein Dimethiconcopolyol-Crosspolymer oder eine beliebige Kombination davon ist.

7. Die Zusammensetzung nach einem der Ansprüche 1 bis 5, wobei das Siliconelastomer das Reaktionsprodukt eines Polysiloxans mit mindestens zwei freien reaktiven Resten aus Vinyl-, Alkyl- und Epoxidresten und eines Polysiloxans mit einer Si-H-Bindung ist.

8. Die Zusammensetzung nach einem der Ansprüche 1 bis 7, wobei der Wirkstoff zur Haarbehandlung mindestens ein grenzflächenaktives Mittel, ausgewählt aus der Gruppe bestehend aus anionischen, nichtionischen, kationischen, amphoteren, zwitterionischen und ampholytischen grenzflächenaktiven Mitteln, umfasst.

9. Die Zusammensetzung nach einem der Ansprüche 1 bis 7, wobei der Wirkstoff zur Haarbehandlung ein Mittel für eine Haarspülung umfasst.

10. Die Zusammensetzung nach einem der Ansprüche 1 bis 9, wobei der in Wasser dispergierbare oder in Wasser lösliche Ester in einer Menge von etwa 0,1 bis 20 Gew.-%, bezogen auf das Gesamtgewicht der Zusammensetzung, vorhanden ist, wobei der Ester einen HLB-Wert von etwa 8,5 und darüber aufweist.

11. Die Zusammensetzung nach einem der Ansprüche 1 bis 10, wobei der HLB-Wert des in Wasser dispergierbaren oder in Wasser löslichen Esters etwa 10 und darüber beträgt.

12. Die Zusammensetzung nach einem der Ansprüche 1 bis 11, wobei der Ester ausgewählt ist aus der Gruppe bestehend aus PEG-7-Oliveat, PEG-7-Glycerylcocoat, PEG-9-Cocoglycerid, PEG/PPG-8/3-Laurat, PEG/PPG-8/3-Diisostearat, Triglycerinlaurat, Glycereth-26-Trioctanoat, Polyglycerin-3-laurat, Sucroselaurat, Sucrosepalmitat und Sucrosestearat.

13. Die Zusammensetzung nach Anspruch 11, wobei der Ester ausgewählt ist aus der Gruppe bestehend aus PEG-7-Glycerylcocoat, PEG/PPG-8/3-Laurat, PEG/PPG-8/3-Diisostearat, Triglycerinlaurat und Sucrosestearat.

14. Die Zusammensetzung nach einem der Ansprüche 1 bis 13, wobei der in Wasser dispergierbare oder in Wasser lösliche Ester in einer Menge von etwa 0,1 bis etwa 5 Gew.-% vorhanden ist.

15. Die Zusammensetzung nach einem der Ansprüche 1 bis 14, wobei das Siliconelastomer in einer Menge von etwa 0,01 bis etwa 5 Gew.-% der Gesamtzusammensetzung vorhanden ist.

16. Die Zusammensetzung nach einem der Ansprüche 1 bis 15, umfassend etwa 5 bis etwa 30 Gew.-% eines grenzflächenaktiven Mittels, ausgewählt aus der Gruppe bestehend aus aninoischen und nichtionischen grenzflächenaktiven Mitteln.

17. Die Zusammensetzung nach einem der Ansprüche 1 bis 16, wobei die Zusammensetzung ein Shampoo ist.

18. Die Zusammensetzung nach einem der Ansprüche 1 bis 15, umfassend etwa 0,1 bis etwa 20 Gew.-% eines grenzflächenaktiven Mittels, ausgewählt aus der Gruppe bestehend aus kationischen, amphoteren und zwitterionischen grenzflächenaktiven Mitteln.

19. Die Zusammensetzung nach einem der Ansprüche 1 bis 15 oder 18, wobei die Zusammensetzung eine Haarspülung ist.

20. Ein Verfahren zur Haarbehandlung, umfassend:
Auftragen einer Zusammensetzung gemäß einem der Ansprüche 1 bis 19 auf das Haar.

## Revendications

1. Composition de rinçage pour soins des cheveux du type non-émulsion comprenant :
(a) un élastomère de silicone réticulé solide,
(b) un ester dispersable dans l'eau ou soluble dans l'eau choisi parmi les esters de polyéthylène glycol, les esters de polyglycéryle et les esters de saccharose,
(c) au moins un agent actif pour le traitement des cheveux,
(d) au moins environ 20 % en poids d'eau.

2. Composition pour soins des cheveux selon la revendication 1, dans laquelle l'élastomère de silicone solide est présent en une quantité efficace pour déposer une quantité apte à absorber le sébum dudit élastomère de silicone réticulé sur les cheveux ou le cuir chevelu.

3. Composition pour soins des cheveux selon la revendication43 1, dans laquelle l'élastomère de silicone est une poudre.

4. Composition pour soins des cheveux selon l'une quelconque des revendications 1 à 3, dans laquelle l'élastomère de silicone comprend des microsphères.

5. Composition pour soins des cheveux selon l'une quelconque des revendications 1 à 4, dans laquelle l'élastomère de silicone a un poids moléculaire moyen en poids de 1 à 20 millions.

6. Composition selon l'une quelconque des revendications 1 à 5, dans laquelle ledit élastomère de silicone est un diméthicone crosspolymer, un organopolysiloxane, un diméthicone/diméthicone de vinyle crosspolymer, un diméthicone co-polyol crosspolymer ou une combinaison quelconque de ceux-ci.

7. Composition selon l'une quelconque des revendications 1 à 5, dans laquelle l'élastomère de silicone est le produit de réaction d'un polysiloxane ayant au moins deux groupes réactifs libres provenant de groupes vinyle, alkyle et époxyde et d'un polysiloxane ayant une liaison Si-H.

8. Composition selon l'une quelconque des revendications 1 à 7, dans laquelle l'agent actif pour le traitement des cheveux comprend au moins un tensioactif choisi dans le groupe constitué par les tensioactifs anioniques, non ioniques, cationiques, amphotères, zwitterioniques et ampholytiques.

9. Composition selon l'une quelconque des revendications 1 à 7, dans laquelle l'agent actif pour le traitement des cheveux comprend un agent de conditionnement des cheveux.

10. Composition selon l'une quelconque des revendications 1 à 9, dans laquelle l'ester dispersable dans l'eau ou soluble dans l'eau est présent en une quantité d'environ 0,1 à 20 % en poids, sur la base du poids total de la composition, l'ester ayant un système HLB d'environ 8,5 et plus.

11. Composition selon l'une quelconque des revendications 1 à 10, dans laquelle le système HLB de l'ester dispersable dans l'eau ou soluble dans l'eau est d'environ 10 et plus.

12. Composition selon l'une quelconque des revendications 1 à 11, dans laquelle l'ester est choisi dans le groupe constitué par l'olivéate de PEG-7, le glycérylcocoate de PEG-7, le cocoglycéride de PEG-9, le laurate de PEG/PPG-8/3, le diisostéarate de PEG/PPG-8/3, le laurate de triglycérol, le trioctanoate de glycereth-26, le laurate de polyglycérol-3, le laurate de saccharose, le palmitate de saccharose et le stéarate de saccharose.

13. Composition selon la revendication 11, dans laquelle l'ester est choisi dans le groupe constitué par le glycérylcocoate de PEG-7, le laurate de PEG/PPG-8/3, le diisostéarate de PEG/PPG-8/3, le laurate de triglycérol et le stéarate de saccharose.

14. Composition selon l'une quelconque des revendications 1 à 13, dans laquelle l'ester dispersable dans l'eau ou soluble dans l'eau est présent en une quantité d'environ 0,1 à environ 5 % en poids.

15. Composition selon l'une quelconque des revendications 1 à 14, dans laquelle l'élastomère de silicone est présent en une quantité d'environ 0,01 à environ 5 % en poids, en poids de la composition totale.

16. Composition selon l'une quelconque des revendications 1 à 15 comprenant d'environ 5 à environ 30 % en poids d'un tensioactif choisi dans le groupe constitué par les tensioactifs anioniques et non ioniques.

17. Composition selon l'une quelconque des revendications 1 à 16, dans laquelle la composition est un shampooing.

18. Composition selon l'une quelconque des revendications 1 à 15 comprenant d'environ 0,1 à environ 20 % en poids d'un tensioactif choisi dans le groupe constitué par les tensioactifs cationiques, amphotères et zwitterioniques.

19. Composition selon l'une quelconque des revendications 1 à 15 ou 18, dans laquelle la composition est un conditionneur de cheveux.

20. Procédé de traitement des cheveux comprenant :
l'application sur les cheveux d'une composition selon l'une quelconque des revendications 1 à 19.
